Europäisches Patentamt

European Patent Office

Office européen des brevets

(11) Publication number: **0 417 301 A1**

# EUROPEAN PATENT APPLICATION
## published in accordance with Art. 158(3) EPC

(21) Application number: 90902817.7

(22) Date of filing: 09.02.90

(86) International application number:
PCT/JP90/00168

(87) International publication number:
WO 90/09590 (23.08.90 90/20)

(51) Int. Cl.⁵: **G01N 33/543, G01N 33/553**

(30) Priority: 10.02.89 JP 29718/89

(43) Date of publication of application:
20.03.91 Bulletin 91/12

(84) Designated Contracting States:
**CH DE FR GB LI NL SE**

(71) Applicant: SHINO-TEST CORPORATION
10, Ichibancho Chiyoda-ku
Tokyo(JP)

(72) Inventor: SUZUKI, Takashi
6295, Tana Sagamihara-shi
Kanagawa 229(JP)
Inventor: HAMAOKA, Akira
11-13, Misono 1-chome Sagamihara-shi
Kanagawa 228(JP)
Inventor: NAITO, Masahiro
7-1-407, Higashinaruse 4-chome Isehara-shi
Kanagawa 259-11(JP)

(74) Representative: Myerscough, Philip Boyd et al
J.A.Kemp & Co. 14, South Square Gray's Inn
London, WC1R 5EU(GB)

(54) **METHOD FOR ASSAYING INDIRECT AGGLUTINATION.**

(57) This invention relates to a method for assaying indirect agglutination such as the microtiter technique for assaying antigen or antibody, wherein magnetized particles are used as a particulate carrier and a magnetic force of a suitable strength is applied to the reaction system for a predetermined period of time. This method takes only about five minutes to conduct an assay which has taken one to two hours heretofore.

# METHOD FOR ASSAYING INDIRECT AGGLUTINATION

## 1. Field of the Invention

This invention relates to an immunological method for determining agglutination using magnetic particles.

## 2. Background of the Invention

In particle agglutination reactions utilizing an immunological reaction, indirect agglutination such as a microtiter method has widely been used in the field of immunological detections because of being inexpensive, simple and convenient. This method detects an immunological reaction of an antigen or antibody, which are physically or chemically bound to the surface of red blood cells, macromolecular particles, gelatin particles, and the like, with an antibody or antigen present in a sample, on the basis of the agglutination on the bottom of a measuring container such as a microplate. This method is excellent in that the measurement can be carried out using only a trace amount of sample and many different samples can be tested simultaneously.

However, as this method determines the agglutination of an antigen-antibody reaction based on the difference between agglutination patterns of particles on the bottom of the measuring container, the measuring period greatly depends on the precipitation rate of the particles. For this reason, if an immobilized red blood cell of an animal such as sheep is used, it requires 2 to 3 hours for measurement due to relatively low specific gravity and small particle size. As described above, there have been developed several methods in which high specific gravity particles such as those of inorganic compounds (Japanese Patent Laid-Open to Public No. Sho 62(1987)-115366) or polyacrolein particles (Japanese Patent Laid-Open to Public No. Sho 63(1988)-24163) are used as a carrier for the measurement in order to reduce the time required.

The measuring period can be reduced to some extent by these methods. However, there is a limitation in shortening the time by utilizing spontaneous precipitation by gravity. Namely, the steeper the slant of the container for the purpose of promoting the sedimentation of particles, or the greater specific gravity or particle size for the purpose of promoting the precipitation as in the methods described above, the more the influence of gravity on the agglutination is increased. This results in an unstable pattern of agglutination which leads to decreased sensitivity and stability although the measuring period can be shortened.

In the light of the situation described above, we have studied for the purpose of providing an indirect agglutination method which can dramatically shorten the measuring period without decreasing the sensitivity and the stability of the measurement, and with which can be measured all kinds of substances as long as the analytes (substances to be measured) are measurable in the art. Thus, we have found that consistent results of measuring the agglutination pattern can be obtained in a very short time with high sensitivity by using magnetic particles bound to a substance having affinity to the analyte, and applying a magnetic force to the magnetic particles.

## 3. Summary of the Invention

This invention relates to a method of determining indirect agglutionation between an analyte and carrier particles having thereon a substance having affinity to the analyte, characterized by binding the analyte to the magnetic particles and by applying, at the same time or subsequently, a magnetic force to the magnetic particles for the measurement of agglutination.

## 4. Detailed Explanation of the Invention

The magnetic particles which can be used in this invention include magnetic substances themselves such as iron, cobalt, nickel, magnetite and ferromagnetic alloys; magnetic particles in which the surfaces of the magnetic particles are coated with polystyrene, silica gel, gelatin, acrylamide, and the like; or particles in which the surface of the particle carrier such as polystyrene, silica gel and the like is coated with a magnetic substance in physicochomical methods or biochemical methods utilizing an immunological reaction.

The size and the specific gravity of particle carriers may be similar to the known particle carriers for the indirect agglutination which have hitherto been used in the art. The particle size may be in the range of 0.01 to 100 microns, preferably 0.5 to 10.0 microns. The specific gravity is not more than 10, preferably not more than 2. Further, it is generally preferable to use those particles which are not so largely affected through precipitation by gravity, in view of the sensitivity of measurement and the stability of agglutination patterns.

As for such particles, the solid carriers which have been used in the art for magnetic separation for enzyme immunoassay, fluoroimmunoassay, chemiluminescence immunoassay, radioimmunoassay, and the like, or those which are used for the carrier for separating cells can be used without any restriction [Japanese Patent Laid-Opens Sho 60(1985)-1564, and Sho 60(1985)-79266, WO-840203, and J. Immunol. Methods, 90, 179(1986), and the like].

The term "indirect agglutination" used herein means the known method to agglutination patterns of the particles at the bottom of a measuring container such as a test tube or a microplate, said agglutination being formed between the particles having the substance which has affinity to the analyte and is physically or chemically bound to the surface of red blood cells, macromolecular particles, gelatin particles and the like.

Any well-known method may be applied to bind the substance having affinity to the analyte in the sample to the magnetic particles. For example, physicochemical method such as a hydrophobic adsoprtion or a covalent bonding may be used. Furthermore, if necessary, the surfaces of the magnetic particles may be treated with a masking agent such as bovine serum albumin casein, or gelatin.

The diluents or the dispersants for use in the measurement may be those which are generally used in indirect agglutination reaction. For example, physiological saline, various buffers, albumin solution, various animal serum solution and various solutions containing surfactants, systhetic macromolecular substances or a combination thereof may be used.

The measuring containers which can be used in this invention are not restricted in their types, materials, shapes, and the like, as long as they can generally be used in the agglutination reaction. For example, test tubes or microplates made of glass, polystyrene, vinyl chloride, and polymethacrylate, can be used. Also applicable is the meauring container disclosed in our pending Japanese Patent Application Sho 62-226561, to which an antigen or an antibody against the analyte is coated.

As the source of the magnetic force in order to precipitate the magnetic particles, a permanent magnet or an electromagnet, etc may be used, with the material, the shape and the apparatus thereof not being specially limited. The extent of magnetic force and the period applied, which vary depending upon the size and the specific gravity of the magnetic particles, a shape of the measuring container, the measurement sensitivity, the period of analysis and the like, may appropriately be selected from among the conditions most suitable for the analysis.

The analyte in this invention is not necessarily limited to specific substance as long as it can generally be measuered in the art. Typical examples of them include CRP, alpha-fetoprotein, CEA, RA, anti-syphilis antibodies, anti-mycoplasama antibodies, FDP, albumin, hepatitis B virus antigen, anti-hepatitis B virus antibodies, anti-HIV antibodies and the like.

The substances having affinity to the analyte are antigens or antibodies against the analyte, receptors such as virus receptors, and hormone receptors, and lectins etc.

The method of this invention which is characterized by using magnetic particles in the agglutination and applying a magnetic force thereon is able not only to increase the rate of precipitating the particles thereby shortening dramatically the time required for the masurement from several hours to several minutes, but also to improve the prior drawbacks of decreases in the sensitivity of measurement and the stabilies of agglutination patterns due to the high specific gravity particles, by limiting the duration of applying the magnetic force.

Now, this invention will be explained with reference to the working examples, which are never intended to limit this invention.

Example 1

Measurement of Human Albumin

(1) Preparation of Magnetic Particles bound to Anti-human Serum Albunmin Antibody

3

To 1 part of (1%[W/V]) magnetic particles made of polystyrene (Dynal Corp, 4.5microns, D: 1.5) suspended in 100mM tris-HCl buffer (pH 8.0), 1 part of a commercially available rabbit anti-human serum albumin antibody(1mg/ml Daco Corp.) was added, and the mixture was incubated at room temperature for 3 hours. After the mixture was washed twice with tris-HCl buffer, the mixture was suspended in 1 part of a tris-HCl buffer containing 5%(W/V) of bovine serum albumin(BSA), and the mixture was incubated at 4°C overnight. After the mixture was washed twice with 100mM tris-HCl buffer, the mixture was suspended in the same buffer containing 1%(V/V) normal rabbit serum to a final concentration of 0.1%-(W/V), resulting in magnetic particles bound to anti-human serum albunmin antibody.

(2) Preparation of the Magnetic Particles Precipitating Apparatus

Ninety-six (96) permanent disc type magnets with 5mm in diameter and 2mm in height (1,300gauss, Pip Fujimoto Corp.) were fixed on a plastic flat plate with an adhesive at the same distance as each well of a 96 well U bottom microplate with N poles upwardly arranged, to make an apparatus for the precipitation of the magnetic particles.

(3) Measurement of Antigen

A purified human serum albumin obtained from normal human serum albumin by salting-out and ion-exchange chromatography according to the conventional manner was adjusted to the concentration of 100$\mu$ g/ml with 100mM tris-HCl buffer (pH 8.0). 25$\mu$ l of this human serum albumin solution was diluted in each of wells of 96 well U-bottom microplate (Kotobuki Glass Corp.) by doubling dilution using 25$\mu$ l each of 100mM tris-HCl buffer containing 1%(W/V) normal rabbit serum and 1%(W/V) of gum arabic. Then, to each of the wells, 25$\mu$ l of 0.1(W/V) magnetic particles bound to anti-human serum albunmin antibody as prepared above was added, and the microplate was shaken by a micromixer for 1 minute. This microplate was placed on the magnetic particles precipitation apparatus described above at a distance of 15mm and left to stand, and the agglutination pattern was read every minute. In this case, a suspension of magnetic particles without anti-human serum albunmin antibody was used as a control.

The measurement could be done in about 10 minutes as shown in Table 1.

## Table 1

| | Time (min.) | Dilution | | | | | | | |
|---|---|---|---|---|---|---|---|---|---|
| | | 2 | 4 | 8 | 16 | 32 | 64 | 128 | 256 |
| HSA-bound magnetic particles | 1 | + | + | + | + | + | + | ± | ± |
| | 2 | + | + | + | + | + | + | ± | ± |
| | 5 | + | + | + | + | + | + | − | − |
| | 10 | + | + | + | + | + | ± | − | − |
| | 20 | + | + | + | + | + | ± | − | − |
| | 30 | + | + | + | + | + | ± | − | − |
| Control magnetic particles | 10 | − | − | − | − | − | − | − | − |
| | 30 | − | − | − | − | − | − | − | − |

+ ······Complete Agglutination, ± ······ Incomplete Agglutination,

− ······ Non-agglutination.

Example 2

Measurement of HBs Antibody

(1) Preparation of HBs Antigen-bound Magnetic Particles

To 1 part of (1%[W/V]) magnetic particles made of polystyrene (Dynal Corp) suspended in 100mM tris-HCl buffer (pH 8.0), 1 part of purified HBs antigen (20ug/ml, Meiji Nyugyo Corp.) was added, and the mixture was incubated at room temperature for 3 hours. After the mixture was washed twice with 100mM tris-HCl buffer, the mixture was suspended in 1 part of the same buffer containing 1% of casein, and the mixture was incubated at 4°C overnight. After the mixture was washed twice with the same buffer, the mixture was suspended in the same buffer to the final concentration of 0.1%(W/V), to give HBs antigen-bound magnetic particles.

(2) Preparation of Measuring container coated with HBs Antigen

50μl of a purified HBs antigen solution which had been diluted to the final concentration of 2μg/ml with PBS was dispensed to each well of a 96-well, V-bottom microplate (Nunc Corp.). After the antigen solution was left to stand for 3 hours at 37°C, it was washed with PBS. 200 μl of 1%(W/V) BSA-PBS solution was added to the antigen solution, and then the mixture was left to stand at 4°C overnight. The HBs antigen coated-microplate was used after washing with purified water.

(3) Measurement of Antibody

Three samples (A, B, and C) of HBs antibody positive human sera and two samples (D and E) of negative sera, both confirmed by a commercially available HBs antibody detecting agent (Fuji Revio Co., Ltd.), were diluted 1:5 with purified water. 25μl of the diluted sera were diluted in the wells of the HBs antigen-coated microplate by doubling dilution using 25μl each of 1000M tris-hydrochloric acid buffer (pH 8.0) containing 0.5%(W/V) of casein. Then, to each of the wells, 25μl of 0.1(W/V) of the above-mentioned suspension of the HBs antibody-bound magnetic particles was added, and the microplate was shaken by a micromixer for 1 minute. This microplate was placed on the magnetic particles precipitation apparatus described in Example 1 at a distance of 5mm and left to stand, and the agglutination pattern was read every minute. The determination with conventional apparatus and the determination using the magnetic particles with no HBs antigen were used as a control.

The results are shown in Table 2. When no magnetic force was applied, it took 2 hours to analyze, whereas in case of the magnetic force was applied under the above-mentioned conditions, the analysis could be effected within approximately 5 minutes. We could not confirm any differences in the sensitivity of the measurements and specifities between both cases, assured us that this invention is more useful.

Table 2

| Sample | Time (min) | | Results |
| Serum | Without magnetic power | With magnetic power | |
|---|---|---|---|
| A | 120 | 5 | Positive |
| B | 120 | 5 | Positive |
| C | 120 | 5 | Positive |
| D | 120 | 5 | Negative |
| E | 120 | 5 | Negative |

Example 3

Measurement of CPR

(1) Preparation of anti-CRP Antibody-bound Magnetic Particles

To 1 Part of magnetic particles (1%[W/V]) (Dynal Corp) suspended in 100mM tris-HCl buffer (pH 8.0), 1 part of purified anti-CRP rabbit antigen (100ug/ml, Biotest Corp.) was added, and the mixture was incubated at room temperature for 3 hours. After the mixture was washed twice with the same buffer, the mixture was suspended in 1 part of the same buffer containing 1% of casein, and incubated at 37°C for 3 hours. After the mixture washed twice with tris-hydrochloric acid buffer, this was suspended in the same buffer containing 0.1%(W/V) normal rabbit serum to the final concentration of 0.1%(W/V), to give anti-CRP antibody-bound magnetic particles.

(2) Preparation of Measuring container coated with anti-CRP Antibody

50μ l each of a purified CRP rabbit antibody (100ng/ml) was dispensed to each well of a 96 well, U-bottom microplate (Nunc Corp.). After the antibody was left to stand for 3 hours at 37°C, it was washed with PBS. 200 μ l of 1%(W/V) BSA-PBS solution was added to the antibody, and then the mixture was left to stand at 4°C overnight, to afford an anti-CRP antibody coated-microplate.

(3) Measurement of Antigen

A purified CRP (Jikken seibutsu Igaku Kenkyusho Corp.) antigen was diluted with 100mM tris-hydrochloric acid buffer (pH 8.0) containing 1mM calcium chloride and 1%(W/V) BSA to a final concentration of 1 μ g/ml. 25μ l of this purified CRP sample was diluted in the wells of the anti-CRP antibody-coated microplate by doubling dilution with 25μ l each of 100mM tris-HCl buffer (pH 8.0) containing 0.5%(W/V) of casein. Then, to each of the wells, 25μ l of 0.1(W/V) of the above-mentioned suspension of the anti-CRP antibody-bound magnetic particles was added, and the microplate was shaken with a micromixer for 1 minute. This plate was placed on the magnetic particles precipitation apparatus described in Example 1 at a distance of 2mm and left to stand, and the agglutination pattern was read every minute. The solution containing no CRP was used as a control.

The results are shown in Table 3. A clear pattern with high specificity could be obtained after about 5 minutes.

## Table 3

| | Time | Dilution | | | | | | | | | |
|---|---|---|---|---|---|---|---|---|---|---|---|
| | (min.) | 2 | 4 | 8 | 16 | 32 | 64 | 128 | 256 | 512 | 1024 |
| Purified | 1 | + | + | + | + | + | + | + | ± | ± | ± |
| CPR | 2 | + | + | + | + | + | + | + | ± | ± | − |
| Sample | 5 | + | + | + | + | + | + | + | ± | − | − |
| | 10 | + | + | + | + | + | + | + | ± | − | − |
| Control | 5 | − | − | − | − | − | − | − | − | − | − |
| | 10 | − | − | − | − | − | − | − | − | − | − |

+ ······Complete Agglutination, ± ······Imcomplete Agglutination,

− ······Non-agglutination.

Example 4

Measurement of HBs Antigen

(1) Preparation of Anti-HBs Antibody-bound Magnetic Particles

To 1 Part of magnetic particles (2%[W/V]) (Dynal Corp) suspended in purified water, 1 part of purified mouse monoclonal anti-HBs antibody (Shinotest Corp.,100ug/ml) was added, and the mixture was incubated at 37°C for 3 hours. After the mixture was washed with PBS, it was suspended in 4 parts of 50mM tris-HCl buffer (pH 8.0) containing 0.5%(W/V) of casein, and the mixture was incubated at 37°C for 5 hours. After the mixture was washed twice with the same solution, it was suspended in 50mM tris-HCl buffer containing 0.25%(W/V) of casein and 200mM sodium chloride to the final concentration of 0.1%(W/V) to give anti-HBs antibody-bound magnetic particles.

(2) Preparation of Measuring container coated with Anti-HBs Antibody

50μl each of a purified mouse monoclonal anti-HBs antibody adjusted to 5μg/ml with PBS was dispensed to each well of a 96 well, U-shaped microplate (Nunc Corp.). After the mouse monoclonal anti-HBs antibody was left to stand for 3 hours at 37°C, it was washed with PBS. 300μl of 50mM tris-HCl buffer (pH 8.0) containing 0.5%(W/V) of casein was added to the mouse monoclonal anti-HBs antibody and then the mixture was left to stand at 4°C overnight, to give an anti-HBs antibody coated-microplate.

(3) Measurement of Antigen

A purified HBs antigen(Meiji Nyugyo Co Ltd.) was diluted to 50ng/ml with PBS. 25μl of this antigen solution was diluted in the wells of the HBs antibody-coated, U-bottom microplate by doubling dilution using 25μl each of 50mM tris-HCl buffer containing 0.25%(W/V) of casein and 200mM sodium chloride. Then, to each of the wells, 25μl of 0.1(W/V) of the above-mentioned suspension of the anti-HBs antibody-bound magnetic particles was added, and the microplate was shaken with a micromixer for 1 minute, and left to stand. This microplate was placed on the magnetic particles precipitation apparatus described in Example 1 at a distance of 2mm and left to stand, and the agglutination pattern was read every minute. Samples containing PBS in place of HBs antigen were used as a contorl.

The results are shown in Table 4. A high detection sensibility and a clear agglutination image could be obtained.

Comparative Example

Measurement of HBs Antigen

(1) Preparation of Immobilized Sheep Red blood Cell coated with Anti-HBs Antibody

To 4 parts of sheep red blood cells (5%[V/V]) suspended in PBS (pH 7.6), 1 Part of 2.5%(W/V) glutaraldehyde in PBS was added, and the mixture was incubated for 2 hours at room temperature. After the incubation, the mixture was washed with PBS, resulting in an immobilized sheep red blood cell. To 1 part of 5% of this immobilized sheep red blood cell suspension, 1 part of 0.005%(W/V) tannic acid in PBS was added, and the mixture was incubated for 30 minutes at room temperature. After the mixture was washed with PBS, a tannic acid-treated immobilized sheep red blood cell was obtained. To 1 part of a 5% suspension of this red blood cell in PBS, 1 part of mouse monoclonal anti-HBs antibody (Shinotest Corp, 2.5mg/ml) was added. The mixture was incubated for 3 hours at room temperature. After incubation, the mixture was washed with PBS and suspended in PBS containing 1%(V/V) normal rabbit serum to a final concentration of 0.5%(W/V) of anti-HBs antibody-bound immobilized sheep red blood cell.

(2) Measurement of Antigen

Purified HBs antigen(Meiji Nyugyo Co Ltd.) was diluted to 50ng/ml with PBS. 25μl of this antigen solution was diluted in the wells of the HBs antibody-coated 96 well U bottom microplate by doubling dilution using 25μl each of PBS solution containing 1%(V/V) of nornal rabbit serum. Then 25μl of 0.5%-(W/V) of anti-HBs antibody-bound immobilized sheep red blood cell described above was added to each well. The microplate was shaken with a micromixer for 1 minute, and left to stand, and then the agglutination pattern was read every minute. Samples containing only PBS in place of HBs antigen solution were used as a control.

The results are shown in Table 4 in comparison with those in Example 4. The measurement by spontaneous precipitation using immobilized red blood cells in conventional methods in the art required 2 to 3 hours for the analysis of the agglutination pattern, whereas the analysis according to the present invention can be done with high sensitivity and clear agglutination pattern within approximately 5

7

minutes.

**Table 4**

| Method | Time (min.) | 2 | 4 | 8 | 16 | 32 | 64 | 128 | 256 | 512 | 1024 | 2048 | 4096 | PBS alone |
|---|---|---|---|---|---|---|---|---|---|---|---|---|---|---|
| | | | | | | | | Dilution of HBs antigen | | | | | | |
| Present invention using magnetic particles | 1 | + | + | + | + | + | + | ± | ± | ± | ± | ± | ± | ± |
| | 2 | + | + | + | + | + | + | ± | ± | ± | - | - | - | - |
| | 5 | + | + | + | + | + | + | ± | ± | - | - | - | - | - |
| | 10 | + | + | + | + | + | + | ± | ± | - | - | - | - | - |
| Conventional method using immobilized sheep red cell | 10 | X | X | X | X | X | X | X | X | X | X | X | X | X |
| | 30 | X | X | X | X | X | X | X | X | X | X | X | X | X |
| | 60 | + | + | + | ± | ± | ± | ± | ± | ± | ± | ± | ± | ± |
| | 120 | + | + | + | ± | ± | ± | ± | - | - | - | - | - | - |
| | 180 | + | + | + | ± | ± | ± | - | - | - | - | - | - | - |

+……Complete Agglutination, ±……Incomplete Agglutination, -……Non-agglutination,

X……Not Determinable

## Claims

1. A method of determining indirect agglutination between an analyte and a magnetic particle having thereon a substance having affinity to the analyte, characterized by binding the analyte to the magnetic particle and by applying, at the same time or subsequently, magnetic force to the magnetic particle for the measurement of agglutination.

2. The method according to claim 1, wherein the magnetic particles comprise iron, cobalt, nickel, magnetite, or a ferromagnetic alloy.

3. The method according to claim 1, wherein the magnetic particles comprise those in which the surface of the magnetic particles is coated with polystyrene, silica gel, gelatin, or acrylamide.

4. The method according to claim 1, wherein the magnetic particles comprise those in which the surface of polystyrene or silica is coated with magnetic substance.

5. The method according to claim 1, wherein the particle size of the magnetic particles is in the range of 0.01 to 100 microns, preferably 0.5 to 10.0 microns.

6. The method according to claim 1, wherein the specific gravity of the magnetic particles is not more than 10, preferably not more than 2.

7. The method according to claim 1, wherein the substance having affinity to the analyte is selected from the group consisting of antigens, antibodies, receptors and lectins.

8. The method according to claim 1, wherein the source of the magnetic force comprises a permanent magnet or an electromagnet.

9. The method according to claim 1, wherein the analyte is CRP, alphs fetoprotein, CEA, RA, anti-syphilis antibody, anti-mycoplasma antibody, FDP, albumin, hepatitis B virus antigen or anti-HIV antibody.

10. The method according to claim 1, wherein the measuring container used for measuring agglutination comprises a test tube or a microplate made of glass, polystyrene, vinyl chloride, or polymethacrylate.

11. The method according to claim 10, wherein the container is coated with a substance having affinity to an analyte.

9

# INTERNATIONAL SEARCH REPORT

International Application No  PCT/JP90/00168

**I. CLASSIFICATION OF SUBJECT MATTER** (if several classification symbols apply, indicate all) [6]

According to International Patent Classification (IPC) or to both National Classification and IPC

Int. Cl[5]  G01N33/543, G01N33/553

**II. FIELDS SEARCHED**

| Minimum Documentation Searched [7] | |
|---|---|
| Classification System | Classification Symbols |
| IPC | G01N33/543, G01N33/553 |

Documentation Searched other than Minimum Documentation
to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| X | JP, A, 60-177265 (Japan Synthetic Rubber Co., Ltd.), 11 September 1985 (11. 09. 85), (Family: none) | 1 - 11 |
| X | JP, A, 59-195161 (Fuji Rebio Kabushiki Kaisha), 6 November 1984 (06. 11. 84), (Family: none) | 1 - 11 |

* Special categories of cited documents: [10]
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art
"&" document member of the same patent family

**IV. CERTIFICATION**

| Date of the Actual Completion of the International Search | Date of Mailing of this International Search Report |
|---|---|
| March 28, 1990 (28. 03. 90) | April 9, 1990 (09. 04. 90) |
| International Searching Authority | Signature of Authorized Officer |
| Japanese Patent Office | |

Form PCT ISA 210 (second sheet) (January 1985)